## Europäisches Patentamt

## European Patent Office

## Office européen des brevets

(11) Publication number: **0 140 713**
**A2**

(12) # EUROPEAN PATENT APPLICATION

(21) Application number: **84307522.7**

(22) Date of filing: **30.10.84**

(51) Int. Cl.⁴: **C 12 P 13/22**
**// C12N9/88**

(30) Priority: **31.10.83 US 547140**

(43) Date of publication of application: **08.05.85**
**Bulletin 85/19**

(84) Designated Contracting States: **DE FR GB IT**

(71) Applicant: **GENEX CORPORATION, 6110 Executive Boulevard, Rockville Maryland 20852 (US)**

(72) Inventor: **Schruber, Jeffrey John, 6200 Meadow Court, Rockville Maryland 20852 (US)**
Inventor: **Vollmer, Patricia Jean, 18928 Clover Hill Lane, Olney Maryland 20832 (US)**
Inventor: **Montgomery, John Patrick, 12512 Piedmont Road, Clarksburg Maryland 29878 (US)**

(74) Representative: **Hildyard, Edward Martin et al, Frank B. Dehn & Co. European Patent Attorneys Imperial House 15-19 Kingsway, London WC2B 6UZ (GB)**

(54) Method for production and recovery of L-Phenylalanine.

(57) A method for the production of L-phenylalanine which comprises combining t-cinnamic acid and ammonium ions in the presence of phenylalanine ammonia-lyase under L-phenylalanine-producing conditions and recovering the L-phenylalanine from the bioreaction mixture so produced characterised in that the source of ammonium ions comprises ammonium carbamate.

## METHOD FOR THE PRODUCTION AND
## RECOVERY OF L-PHENYLALANINE

The present invention relates to an improved method for producing L-phenylalanine. More particularly, the invention relates to an improvement in the production of L-phenylalanine by the phenylalanine ammonia-lyase (PAL)-catalyzed reaction of t-cinnamic acid and ammonium ions.

L-Phenylalanine is an essential amino acid in man, and therefore is an important component of parenteral and enteral nutrition products. In addition, L-phenylalanine can be used as a starting material for the production of important chemicals, such as the dipeptide sweetener, aspartame. Various microbial processes for the production of L-phenylalanine are known. For example, U.S. Patent 3,660,235 describes the production of L-phenylalanine by L-phenylalanine analog-resistant strains of Brevibacterium, Corynebacterium, Arthrobacter, Bacillus and Candida. Production methods for this amino acid by tyrosine-requiring mutants of certain strains of Brevibacterium, Corynebacterium, Arthrobacter, and Escherichia are also known. (see U.S. Patent 3,654,079 and 3,909,353).

Phenylalanine ammonia-lyase catalyzes the breakdown of L-phenylalanine to t-cinnamic acid and ammonia. This enzymatic reaction is reversible, and British Patent 1,489,468 discloses a process for the production of L-phenylalanine which involves the PAL-catalyzed reaction of t-cinnamic acid with ammonium ions to yield L-phenylalanine. This reaction has been found to be useful for the commercial production of L-phenylalanine, and there is a continuing need to adapt the overall process to the commercial production setting. In this regard, there is a particular need to improve upon the recovery of L-phenylalanine from bioreaction mixtures.

In the aforementioned British Patent, 1,489,468 L-phenylalanine is initially recovered by precipitating it at its isoelectric point (pH 5.5), or alternatively, absorbing the L-phenylalanine on a cation exchange resin at a low pH, followed by elution with ammonium hydroxide at a high pH. The L-phenylalanine is subsequently freed from the ammonium ion by heating the solution. That patent also discloses a recovery procedure which involves converting the L-phenylalanine to the methyl ester _in situ_ and crystallizing it as the hydrochloride salt. The recovery procedures described in British Patent 1,489,468 have not been found totally satisfactory for commercial scale operations. Simply precipitating the L-phenylalanine at its isoelectric results in less than ideal yields and product purities. Although ion exchange chromatography can produce a relatively pure product, it is not convenient for large scale operations.

Yamada, S. et al., Applied and Environmental Microbiology, November, 1981, pp. 773-778, described laboratory procedures for the production of L-phenylalanine by the PAL-catalyzed reaction of t-cinnamic acid in ammonium ions. In the procedure described in that reference, the L-phenylalanine was recovered from a cell-containing bioreaction mixture by a multi-step process which included centrifugation to remove the cells and solid material, removal of excess ammonia from the supernatant _in vacuo_, adjustment of the pH to 1.8 to precipitate t-cinnamic acid (which was then removed by filtration), cation exchange chromatography to absorb the L-phenylalanine, and elution of the L-phenylalanine with a base. The authors reported a 69% percent yield using this procedure. Although the phenylalanine produced by the procedure was quite pure, as indicated above, the column chromatography step is disadvantageous for large scale production.

Accordingly, there is a continuing need to improve upon the procedures for recovering L-phenylalanine from bioreaction mixtures resulting from the PAL-catalyzed production of L-phenylalanine.

According to the present invention we provide a method for the production of L-phenylalanine which comprises combining t-cinnamic acid and ammonium ions in the presence of phenylalanine ammonia-lyase under L-phenylalanine-producing conditions and recovering the L-phenylalanine from the bioreaction mixture so produced characterised in

that the source of ammonium ions comprises ammonium carbamate.

In the method of the present invention, microbially produced phenylalanine ammonia-lyase is contacted with t-cinnamic acid (which is effectively ionised to t-cinnamate ions at the operating pH) and ammonium ions to produce L-phenylalanine. The phenylalanine ammonia-lyase may be present in a microbial cell-containing aqueous broth, the separated cells therefrom, or as the isolated enzyme. In addition, the whole cells or isolated enzyme may be immobilized on a solid support. The PAL is produced by cultivating a PAL-producing microorganism in a suitable nutrient medium. Microbial strains that produce PAL and the microbiological process employing those strains are well known, and are described, for example, in the British Patent and the article by Yamada referred to above and also in our GB-A-2,127,821 and United States Patent Applications Serial No. 547,129, filed October 31, 1983 and Serial No. 547,139, filed October 31, 1983 corresponding to our copending EP-A-     and EP-A respectively. Copies of the aforementioned European Patent Applications are available on the public file of the present Application.

The enzymatic reaction is generally conducted by contacting a substrate solution with the PAL. Prior to this invention, the substrate solution has typically been prepared by dissolving t-cinnamic acid in a concentrated ammonium hydroxide solution. Water and a mineral acid have then been added to adjust the substrate volume and pH, respectively. Alternatively, the ammonium ions have been provided to the substrate solution in the form of an ammonium salt, such as ammonium chloride. The aforementioned U.S. Patent Application Serial No. 432,182 describes that halide ions have a deleterious effect on phenylalanine ammonia-lyase, and therefore teaches the use of substantially halogen free substrate solutions. Thus, it is taught that preferred ammonium salts include ammonium sulfate, ammonium nitrate, ammonium citrate, ammonium acetate and ammonium phosphate.

The present invention involves the use of ammonium carbonate as a source of ammonium ion in the substrate solution. In general, the substrate solution can be prepared by combining ammonia, in the gaseous form or as ammonium hydroxide with ammonium carbonate to provide the desired ammonium carbonate concentration. An alternative and preferred procedure for preparing the substrate solution is to dissolve the t-cinnamic acid in an aqueous ammonia solution, then to bubble carbon dioxide gas through the solution until the desired pH is obtained. The concentration of ammonium ion in this substrate solution advantageously ranges from about .1 mole per liter to about 9.0 moles per liter, preferably from about 5.0 mole per liter to about 8.0 moles per liter. The substrate solution also contains t-cinnamic acid. The concentration of t-cinnamic acid in the solution is controlled such that the enzyme-catalyzed reaction proceeds at a useful rate, yet the concentration is not so high as to cause substrate inhibition of the enzyme activity. In general, the concentration of t-cinnamic acid ranges from about 5 to about 25 g/l, preferably from about 10 to about 20 g/l. The pH is controlled such that phenylalanine producing conditions are maintained when the PAL catalyst is contacted with the substrate solution. This pH is advantageously between about 9 and 11, preferably between about 10.4 and 10.8.

After the PAL has been contacted with the substrate solution for a time sufficient to convert a substantial amount of the t-cinnamic acid to L-phenylalanine, the L-phenylalanine can be recovered and purified. At the pH of the substrate solution, both the t-cinnamic acid and the L-phenylalanine are soluble. Therefore, cells and other solids can be removed by centrifugation or filtration to yield a clarified solution. This solution can then be desalted conveniently by volatilizing ammonia and carbon dioxide resulting from the ammonia and ammonium carbonate initially added to the substrate solution. The ability to remove these ions from the solution by volatilization is a principal advantage of using ammonium carbonate as an ammonium source. An additional important advantage of this process is that ammonia and ammonium carbonate can be recovered for reuse. Recovering these chemicals improves the overall economic efficency of the process and also minimizes the release of these materials into the environment. The desalting operating is generally carried out by heating the clarified solution to a temperature that facilitates volatilization of the ammonium carbonate. Temperatures

ranging from about 10 to 120°C, preferably from about 40 to 60°C, depending on the pressure in the reaction vessel, can be used for this purpose. Vacuum evaporation, air stripping or steam stripping can be used effectively to accelerate the volatilization of the ammonium carbonate to ammonia and carbon dioxide. Vacuum evaporation at temperatures of from about 40°C to about 60°C is a preferred technique. A particularly preferred procedure is to heat the solution to a temperature of about 50°C at an absolute pressure of about 100 mm Hg and remove the ammonia and carbon dioxide by evaporation. Initially, ammonia gas is liberated, and the removal of ammonia causes the pH of the solution to drop. When the pH reaches a certain point, carbon dioxide begins to volatilize which offsets the decrease in pH. Essentially all of the ammonia and carbon dioxide can be effectively removed from the solution using this procedure, if desired, and these gases can be recovered by condensation of the vapors.

Removing the salt from the solution by volatilzation, permits the resulting salt-free solution to be concentrated further to crystalize the L-phenylalanine in high yield.

Crystallization of L-phenylalanine is accomplished by evaporating the solution, e.g., at a temperature of from about 10°C to about 120°C, until the L-phenylalanine concentration is from about 75 to about 300 g/l, preferably about 150 to about 200 g/l. A preferred procedure is to evaporate the solution at reduced pressure at a temperature of from about 20°C to about 70°C until the desired L-phenylalanine concentrations are obtained. During this evaporation, an alkaline pH is maintained to avoid precipitation of any t-cinnamic acid which remains in the solution and to maximize the yield of L-phenylalanine. This pH generally ranges between about 6 and 9, preferably about 7.0-8.5. The resulting L-phenylalanine slurry is then cooled gradually to a temperature which results in substantial crystallization of the L-phenylalanine. The solution is preferably cooled to a temperature of from about -5°C to about 25°C. The resulting L-phenylalanine crystals can then be recovered, e.g., by filtration or centrifugation, washed with cold water and dried. A second crop of crystals can be recovered by further concentrating and cooling the mother liquor, if desired. The mother liquor may also be recycled to a subsequent fermentation as a PAL inducer. Any t-cinnamic acid remaining in the mother liquor can be recovered by acidification and precipitation, and this t-cinnamic acid is advantageously recycled to the bioreactor.

In an alternative embodiment of this invention, it is sometimes advantageous to remove and recover any t-cinnamic acid in the reaction mixture prior to recovery of the L-phenylalanine.

Prior to precipitating the L-phenylalanine, the desalted solution is advantageously acidified with a strong mineral acid, such as sulfuric or hydrochloric acid, to a t-cinnamic acid-precipitating pH. Such pH generally ranges from about 1.0 to about 3.0 preferably from about 1.5 to 2.5. Substantially quantitative precipitation of the t-cinnamic acid can be achieved by cooling the solution to a temperature from about -5°C to about 25°C preferably from about 0° to 5°C. The precipitated cinnamic acid can then be recovered by filtration or centrifugation and if desired, reused.

The resulting solution is then concentrated, e.g., by evaporation under vacuum, until a relatively high L-phenylalanine concentration is achieved. The desired concentration is one which, upon subsequent crystallization, results in substantial precipitation of the L-phenylalanine present in the solution. Preferably, substantially quantitative precipitation of the L-phenylalanine is achieved. To accomplish this goal, the phenylalanine concentration ranges from about 25 to about 150 grams per liter, preferably from about 50 to 150 grams per liter.

Crystallization of the L-phenylalanine from this solution is advantageously done by adjusting the pH of the solution to approximately the isoelectric point of phenylalanine using a base. Preferably ammonia or ammonium hydroxide is used for this pH adjustment. The pH generally ranges from 3.5 to about 6.5. During this pH adjustment, it is preferred that this solution be heated to a temperature which causes the L-phenylalanine to be retained in solution during the pH adjustment. For example, a temperature of from about 40°C to about 100°C can be employed. A gradual cooling of this solution results in crystallization of the L-phenylalanine in high purity. Substantially quantitative precipitation can be achieved by cooling to a temperature of from about -5°C to about +25°C. The L-phenylalanine crystals can then be recovered by filtration or centrifugation, washed with cold water and dried.

The process of this invention has been found particularly suitable for large scale commercial operations. The process yields L-phenylalanine crystals of high purity in good yield.

The invention is further illustrated by the following examples, which are not intended to be limited.

## Example I

This example describes the recovery of L-phenylalanine from a bioreaction medium in accordance with the method of this invention. The bioreaction medium was obtained by reacting t-cinnamic acid with ammonia in the presence of PAL-containing Rhodotorula rubra cells. The enzyme substrate solution was prepared by dissolving t-cinnamic acid in an aqueous ammonia solution and adjusting the pH to 10.4 to 10.8 using carbon dioxide. The final reaction volume was 1236 liters and contained a total of 51.912 kg of L-phenylalanine (42.0 g/l).

This reaction mixture was cooled to a temperature of 10°-15°C to minimize ammonia losses and to inhibit the breakdown of L-phenylalanine. The mixture was centrifuged and then filtered through a pressure leaf filter to remove the cells. At this point, the filtrate (1190 liters) had a phenylalanine concentration of 38.2 g/l. This filtrate was heated to 40°C under vacuum (25" Hg) until concentrated to 485 liters. Ammonia and carbon dioxide were thus removed from the solution and recovered by condensation of the vapors. The pH of the resulting concentrated solution was about 9.5, and the L-phenylalanine concentration was 83.1 g/l. Some particulate matter formed in the solution during concentration and salt removal, and this particulate matter was removed by filtration through a pressure leaf filter prior to further processing. The clarified concentrate was again heated to 40°C under vacuum (25" Hg) and evaporated to a volume of 240 liters. The pH was adjusted to 8.7 and this concentrate was cooled to 5°C. The L-phenylalanine crystals were recovered by centrifugation and washed with cold water. Approximately 53% of the L-phenylalanine originally present in the reactor was recovered in the form of substantially pure crystals. An additional 25% of the L-phenylalanine was present in the mother liquor and wash solutions, and a portion of this material could be recovered in a second crop of crystals if desired.

## Example II

The procedure of Example I was repeated in all essential details. The original reaction mixture volume was 1245 liters, and the mixture had a phenylalanine concentration of 39 g/l. A total of 38.0 kg of L-phenylalanine (78.3%) was recovered in the first batch of crystals recovered from the concentrated, desalted solution. The mother liquor from the phenylalanine crystallization contained 9.0 kg (18.5%) of L-phenylalanine, and this was recycled to a subsequent fermentation as a PAL-inducer. In addition, the mother liquor contained 16.5 kg of t-cinnamic acid which was also recycled to a subsequent bioreaction.

## Example III

This example describes the recovery of L-phenylalnaine from a bioreaction mixture according to an embodiment of the present invention, wherein t-cinnamic acid is recovered prior to L-phenylalanine precipitation.

A bioreaction mixture prepared by reacting t-cinnamic acid with ammonia in the presence of PAL-containing R.rubra cells was centrifuged and filtered to remove the cells. Prior to centrifugation, the mixture had a pH of 10.52, a temperature of 34°C and a volume of 1000 liters. The mixture contained 21.0 g/l L-phenylalanine and 15.3 g/l t-cinnamic acid.

The reaction mixture was centrifuged and filtered to remove the cells, and was transferred to vessels equipped with heating and agitation means. The clarified solution was heated to 90°C and was sparged with air. Ammonia and ammonium carbonate were recovered by condensing the vapors. The air sparging was contained until the volume was about 350-37⁵ liters and the L-phenylalanine concentration was 70-80 g/l. This concentrate was then transferred to another vessel, where the pH was adjusted to 2.0-2.1 with 50% $H_2SO_4$. The acidified solution was cooled to 0°-10°C and was held for four hours. The resulting t-cinnamic acid crystals were removed from the solution by centrifugation. The mother liquor was transferred to another vessel and held at 23-24°C for one hour and 8 kg of filter aid was added. The filter aid and remaining t-cinnamic acid was removed by filtering the solution through a filter press. The volume of the filtrate (including wash solutions) was 270 liters, contained 1.88 g/l t-cinnamic acid and 59.5 g/l L-phenylalanine. This filtrate was transferred equally to two vessels, where the pH was adjusted to 3.5-3.6 with 29% aqueous ammonia and the solution heated to 60-70°C. Vacuum (175 mm Hg) was applied, and the contents were evaporated to a L-phenylalanine concentration of 200 g/l. The concentrated mixture was cooled at 10°C/hour to less than 10°C and was held at that temperature for four hours. The resulting L-phenylalanine crystals were removed by centrifugation, washed with cold deionized water and dried. The mother liquor and wash solutions were pooled with corresponding solutions from another recovery run, and second crop crystals were recovered by concentration and cooling of the mixtures as described above. The yield from the process for the first crop crystals was approximately 70%.

12

## CLAIMS

1. A method for the production of L-phenylalanine which comprises combining t-cinnamic acid and ammonium ions in the presence of phenylalanine ammonia-lyase under L-phenylalanine-producing conditions and recovering the L-phenylalanine from the bioreaction mixture so produced characterised in

that the source of ammonium ions comprises ammonium carbamate.

2. The method of claim 1, wherein the recovery of L-phenylalanine includes the step of removing the ammonium and carbonate ions by volatilization.

3. The method of claim 2, wherein the volatilization of the ammonium and carbonate ions is accomplished by evaporation, air sparging or steam distillation.

4. The method of claim 3, wherein ammonia and ammonium carbonate are recovered for reuse by condensing the vapours from the volatilization and collecting the condensate.

5. The method of any one of claims 2 to 4 wherein the volatilization is accomplished by evaporation or air sparging at a temperature of from about 10°C to about 120°C.

6. The method of claim 5, wherein the volatilization is accomplished by evaporation at reduced pressure at a temperature of from about 40°C to about 60°C.

7. The method of claim 1, wherein the L-phenylalanine is recovered by a process comprising:

    (a) removing the solids from the bioreaction mixture to form a clarified solution;

    (b) removing the ammonium and carbonate ions from the clarified solution by volatilization, to form a desalted solution;

(c) adjusting the pH of the desalted solution to about 6 to about 9;

(d) evaporating the desalted solution until the concentration of the L-phenylalanine ranges from about 75 g/l to about 300 g/l to form a concentrate;

(e) cooling the concentrate to an L-phenylalanine-precipitating temperature, to form a L-phenylalanine slurry; and

(f) removing the L-phenylanine crystals from the L-phenylalanine slurry.

8. The method of claim 7, wherein the volatilization of step (b) is accomplished by evaporation or air sparging at a temperature of from about 10°C to about 120°C.

9. The method of claim 7, wherein the volatilization of step (b) is accomplished by evaporation at reduced pressure at a temperature of from about 40°C to about 60°C.

10. The method of claim 8, wherein, in step (c) the pH is adjusted to a range of from about 7.0 to about 8.5.

11. The method of claim 8, wherein, in step (d), the clarified solution is evaporated at a temperature of from about 40°C to about 60°C under reduced pressure, and the evaporation is continued until the L-phenylalanine concentration ranges from about 150 g/l to about 200 g/l.

12. The method of claim 11, wherein, in step (e) the concentrate is cooled to a temperature of from about -5°C to about 25°C.

13. The method of claim 1, wherein the L-phenylalanine is recovered by a process comprising:

(a) removing the solids from the bioreaction mixture to form a clarified solution;

(b) removing the ammonium and carbonate ions from the clarified solution by volatilization to form a desalted solution;

(c) acidifying the desalted solution to an acidic t-cinnamic acid-precipitating pH which is sufficiently low that the L-phenylalanine remains in solution and removing the precipitated t-cinnamic acid to form a t-cinnamate-free, clarified solution;

(d) crystallizing the L-phenylalanine from the t-cinnamate-free, clarified solution by adjusting the pH to an L-phenylalanine-precipitating pH and cooling the solution to an L-phenylalanine-precipitating temperature; and

(e) separating the L-phenylalanine crystals from their mother liquor.

14. The method of claim 13, wherein the volatilization of step (b) is accomplished by evaporation or air sparging at a temperature of from about 10°C to about 120°C.

15. The method of claim 13, wherein the volatilization of step (b) accomplished by evaporation at reduced pressure at a temperature of from about 40°C to aobut 60°C.

16. The method of claim 14, wherein, in step (c) the t-cinnamic acid-precipitating pH ranges from about 1 to about 3, and in step (d) the L-phenylalanine-precipitating pH ranges from about 3.5 to about 6.5.

17. The method of claim 16, wherein the L-phenylalanine-precipitating temperature ranges from about -5°C to about 25°C.